# EUROPEAN PATENT APPLICATION

(11) **EP 3 822 984 A1**
(43) Date of publication of application: **19.05.2021**
(21) Application number: 20208244.2
(22) Date of filing: 17.11.2020
(51) Int. Cl.: G16H 40/20

(54) **A SYSTEM AND A PROCEDURE FOR NURSE CALL AND MANAGING OF HEALTH CARE AND NURSING**

(30) Priority: 18.11.2019 SI 201900231
(71) Applicant: EUROTRONIK Kranj d.o.o., 4000 Kranj (SI)
(72) Inventor: Gorjanc, Igor, 4000 Kranj (SI); Magdic, Tomi, 4243 Brezje (SI); Krajnc, Andraz, 4000 Kranj (SI)
(74) Representative: Patentni Biro AF d.o.o.

(57) **Abstract**

The present invention belongs to the field of communication techniques and systems adapted for use in health care, more precisely to the field of devices and systems for calling medical personnel and for managing health care.

The essence of the invention is an intelligent nurse call system for hospitals, clinics, nursing homes and other health-nursing institutions, which in one device combines a nurse call system with recording events and management of health care and nursing. The room module is an interactive touch-screen display, which combines nurse calls and documentation as well as management of health care into one procedure. It always enables access to at least the following information:
- staff member logged in the room,
- arrival into the room and time spent in the room,
- performed activities in the room, for example reanimation, temperature measurement, blood pressure measurement, etc., and the person for which the activities were performed.

## Description

### Field of the invention

The present invention belongs to the field of communication techniques and systems adapted for use in health care, more precisely to the field of devices and systems for calling medical personnel and for managing health care. The invention relates to a nurse call system and recording all events in connection with patients as well as to devices forming the system and ensuring its operation. The invention further relates to a procedure of nurse call and management of health care and nursing.

### Background of the invention

Nurse call is a name for a system for controlling, guarding and alarming intended for use in hospitals, health centres, nursing homes or other facilities, where spaces are guarded. The purpose of nurse call systems is efficient calling of medical personnel into a room, where a patient needs help. Usually the patient in a bed triggers a call of the medical personnel into the room where the patient is located. The call is triggered with a button and a rope. A nurse receives on her unit an alarm and information about the room where the call has been made. At the same time a light or a signal above the door or the bed is turned on. When the nurse comes to the room or the patient who made the call turns off the call with a special button or a switch.

Usual solutions of nurse calls are limited to turning off the alarm of the call or at the same time entering the time from the call to the push of the response button at the time of arrival and leaving, which partly enables tracking of medical staff responses. However, these systems do not enable wider use of the nurse call systems as they have no options for entering logs or following other events connected to medical treatment and nursing.

Therefore, the technical problem, which is solved by the present invention, is a construction of a device and a system, which will combine operation of known nurse call systems and upgrade with additional functions, which will contribute to more efficient health care and higher safety of patients or people in nursing homes.

### State of the art

Patent application WO2017131796 discloses a system for nurse calls, which comprises a hosting device and a multifunctional cable for nurse call, which includes a button for calling a nurse. The cable is arranged to be connected to the hosting device and to a mobile device located near the patient. The cable enables power supply to the patient's device, wherein the nurse call may be made with the button, which through the hosting device reaches the medical staff station. This solution is much simpler than the present invention, as it only allows medical staff calls and does not enable other procedures connected with treatment and nursing.

Patent application JP2014042735 describes an electronic device for a nurse call, which can track the response times. This solution allows only limited control of medical care, i.e. only from the view of responsiveness of medical staff.

Document CN204180196 discloses a system, which includes a station for medical staff, mobile units for medical staff, units above beds connected with the station for medical staff, so that a nurse call may be made from any unit above the bed. In addition, the system has a possibility of audio and/or video module, module for measurement of body temperature, blood pressure and/or heart rate, which can show the measured values on mobile units for medical staff. The system allows tracking some of the vital functions of patients; however, this is only related to displaying measured values, which differs from the present invention.

An upgraded solution is described in patent application JP2001243582, which describes a system for managing medical care, wherein a controller for controlling calls and communication between units in hospital rooms and the main unit of the medical staff in a control room and a managing device connected to the controller, wherein the managing device has a part for storing files with information such as patient's data, patient's medical history, care guidelines, nutrition guidelines and similar. Data from measuring devices such as thermometers may be automatically stored in the managing device. The present invention is more complex than this solution, as it allows far more possibilities of control and management of health care and treatment of patients.

### Description of the solution of the technical problem

Technical problem is solved as defined in the independent claim, wherein the preferred solutions are defined in the dependent claims.

The essence of the invention is a nurse call system for hospitals, clinics, nursing homes and other health-nursing institutions, which is based on cutting-edge IP technology and which in one device combines a nurse call system with recording events and management of health care and nursing. Communication in the system is based on an internet protocol and complies to all highest security requirements (among others also to the German standard DIN VDE 0834, 1. and 2. part). The mentioned system comprises the following interconnected devices, units or modules:
- at least one device in a room or any other space for nurse call and management of health care and nursing (i.e. room module),
- at least one user device for installation in a bathroom, above or beside a bed in a room of a healthcare institution, said device having at least a button for calling medical staff (i.e. patient module),
- at least one central control device for installation in a room for medical staff, which tracks calls from patient modules and stores information from the room module to a server of the system,
- at least one device for medical staff, which can connect to the room module and/or the central control device,
- at least one notifying lights for installation above room door,
- at least one suitable interface, which enables connection of all or a part of patient modules installed in any space wherein the interface has enough spaces for input and output signals,
wherein identification and use of the room module and the central control unit enables card identification, code indentification, preferably with PIN numbers, or any other suitable similar identification means; and
wherein the system performs at least the following operations:
- enabling calling of nursing staff from any patient module,
- enabling cancelling of nurse call on the room module, on the device for medical staff and/or on the central control unit,
- identification of staff or other employees on the room module,
- calling a doctor and/or emergency team via the room module or the device for medical staff,
- showing calls and emergency with the lights above doors and the central control device,
- enabling assistance call (=calling additional medical/nursing staff) via the room module or the device for medical staff,
- confirming performed checks,
- entering patient's data, status of the patient and consumed medications, food and/or drink and performed therapy,
- logging events and storing them on the system server,
and the operation of the system is supported with a computer program or protocols, which ensure suitable performance and logging operations as well as communication of all devices included in the system.

The room module also enables log-in of physiotherapists, occupational therapist, medical doctors and other healthcare workers in the same way as for nurses. In addition to the call button a button for cancelling a call may be provided, the said cancel button being suitably connected to the room module via an interface. At the same time the room module can serve as an information point for the patient's family, if suitable access to information has been provided.

In addition, the system may have at least one device for cleaning staff, janitors and other employees of the health institution (so called supporting staff), which is preferably a mobile phone, a smart phone, a tablet or a similar device. Thus, the system additionally performs at least the following operations:
- calling or notifying maintenance staff with a notification about needed repair,
- calling or notifying cleaning staff with a notification about needed cleaning,
- identification of supporting staff on the room module, wherein they cannot access to patient information and can only enter information about maintenance or cleaning,
- entering and storing of performed tasks on the system server.

In addition, the system may also comprise a ceiling display of events connected with the central control device, which in case of a call shows the room number and optionally the name or number of the patient who made the call.

Components of the system are electronically connected with each other, wherein they can communicate via wired networks or wireless networks, especially via Bluetooth network, preferably via so called mesh Bluetooth network.

Patient's module for nurse calls may be a call string at the bedside, a call button or any other suitable unit, wherein it is located above or beside the bed and/or in the bathroom or toilet, respectively. The patient's module may be an independent module such as a pendant or a bracelet, so that patients that are able to move can freely move.

The room module, i.e. the device in the hospital room or any other room, where the system is installed, is essentially an interactive room touchscreen display, which combines the nurse call and whole documentation and management of medical care into one procedure. This room module has its own screen, power supply, processor, module for a wired or a wireless connection, preferably Bluetooth, and other necessary components needed for operation, and is connected to the central control unit and to the system's server. It always enables access to at least the following information:
- staff member logged in the room,
- arrival into the room and time spent in the room,
- performed activities in the room, including performed measurements and results,
- the person for which the activities were performed.

Furthermore, the device also allows electronic logging and management of all performed activities as well as maintenance works (technical malfunctions, cleaning, etc) in the room. Data may be entered directly on the room module or via a device for medical staff, wherein the preferred choice is the room module. Person, who spots a malfunction, enters the latter in the device, and this information is immediately shown on the device for maintenance staff or on a suitable device such as a mobile phone, smart phone, tablet and/or a computer. When the person responsible for addressing the said malfunction responds, the performed activity is immediately logged in the system. Display of open and completed actions is shown, so that maintenance is optimized, while all activities can be easily followed. Operation of the device enables a suitable computer program and/or a mobile application, which performs processes for user identification, entering performed activities and data about the treated person, wherein the program ensures storing time stamps of arrival and departure, which enables tracking of treatment times as well as identification of presence in the room in time of performed activities and possible values of performed measurements, consumed food, drink and/or medication.

The medical staff device is preferably designed as a smart phone, tablet or similar device known in the field, to which a computer program and/or a mobile application can be stored, the said program or application allowing overview of the present staff and setting up calls of staff, wherein the staff may communicate without the need for a SIM card. Said device may communicate with the room module or the central control unit, so that information about performed activities, results of performed measurements, data about food, drink and medications, and similar are entered. The device preferably enables additional functions such as notifications and reminders, which can adapt with regards to the needs of users, which is particularly useful in cases where patients need medication(s) at a particular time. The device for medical staff is thus an upgraded existing DECT phone or pager.

User device of a patient or a person in a room, which is located above or beside the bed, has at least a call button for calling medical staff and an LED indicator of the made call. Optionally, it also has further functional buttons, which enable control of lights above the bed, TV, radio, speech communication with nursing staff, etc. It connects with the room module and/or the central control unit via wired or wireless connection, preferably via Bluetooth, which ensures reliability and control or operation in the same way as for cable connections (in agreement with valid standards for nurse calls, particularly DIN VDE 0834).

The invention may be additionally equipped with various wireless sensors, such as movement sensors, sensors for sensing door opening, air quality sensors, smoke detectors and similar, which are installed in individual rooms, above doors and/or windows and/or above/below beds. Triggering of a sensor can be detected by the room module, which transmits information about the sensor activation to the central control unit for medical staff, so that they can respond. This addition improves safety of patients or people in the room, as living conditions can be regulated, while movement sensors and sensors of door/window opening can prevent or at least indicate possible leaving of the patient, which is of utmost importance for patients, who are not allowed to move or for people suffering from dementia.

Furthermore, the system may also comprise a wireless pendant or a wireless bracelet for patients, so that a call for help of medical staff can be made based on sensing the status of the pendant/bracelet, wherein the bracelet comprises:
- wrist strap with a suitable closure, and
- a transmitter mounted on the said strap with a button for calling help, wherein said transmitter can connect to a central control unit of the system, as well as
- a sensor for sensing wear,
wherein the transmitter preferably also has a locating system provided for determination of patient's location in case he is not in a room. This is particularly useful for people in homes for elderly or health centres without movement limitation. Thus, medical staff can respond in shorter times.
Automatic reset of the call is achieved based on location of the nurse and a patient, so that after a triggered call via the patient module the responsive medical staff has a special emitter emitting a weak signal including identification data about the person wearing the emitter (for example a nurse). Upon reaching the required distance of the medical staff to the patient, the patient's module senses the signal and sends call cancellation to the system. It is important that the weak signal has such strength that the call is reset only when the nurse reaches the patient. Because the signal includes identification data, the system automatically stores data about the responsive staff (identity of the nurse).

The system according to the invention may be installed in existing or new buildings where medical care or patient control is necessary, such as hospitals, health centres, health resorts, and nursing homes for elderly.

The procedure for nurse call and management of medical care and nursing comprises at least the following steps:
- calling medical staff with the button on the patient module, which may also be a bracelet or a pendant,
- alerting medical staff about the call via the central control device and/or the device for medical staff, optionally the call may also be displayed on the display screen,
- lighting of the notifying light above the door of the room, where the call has been made,
- identification of responsible medical staff on the room module for confirming presence with card identification, PIN code or any other known identification method,
- aiding the person who called or any other person, if the calling person has called to help someone else,
- possible call of additional staff, doctor, emergency team, wherein in case of the latter the notifying light emits blue light,
- entering performed activities in the room on the room module and/or device for medical staff,
- overview of possible other activities, particularly maintenance or cleaning,
- logging out on the room module before leaving the room,
- storing data on the server and possible analysis of reponse times, treatment time, performed activities and similar.

Operation of the system is supported with a computer program, which performs at least the following functions:
- Enabling calling of nursing staff from any patient module,
- Enabling cancelling of nurse call on the room module, on the device for medical staff and/or on the central control unit,
- Identification of staff or other employees on the room module,
- Calling a doctor and/or emergency team via the room module or the device for medical staff,
- Showing calls and emergency with the lights above doors and the central control device,
- Enabling assistance call (=calling additional medical/nursing staff) via the room module or the device for medical staff,
- Confirming performed checks,
- Entering patient's data, status of the patient and consumed medications, food and/or drink and performed therapy,
- Logging events and storing them on the system server,
- Full support of operation for room modules and storing information in a database,
- Directing calls with regards to set parameters (time of day, department, call type, etc.) and
- Preferably enables manual or automatic reports of collected data.

In the scope of the invention as described herein and defined in the claims other embodiments of the device and the system for nurse call and management of health care obvious to the person skilled in the art are possible, which does not limit the essence of the invention as described herein and defined in the claims.

The device, system and the procedure for nurse call and management of health care and nursing according to the invention will be disclosed in further detail based on exemplary embodiments and figures, which show:
Figure 1 Scheme of the procedure for nurse call and management of health care and nursing
Figure 2 A possible embodiment of the system in case of IP connection
Figure 3 The preferred embodiment of the display of options on the room module
Figure 4 A possible embodiment of the patient's module shaped as a bracelet and as a pendant

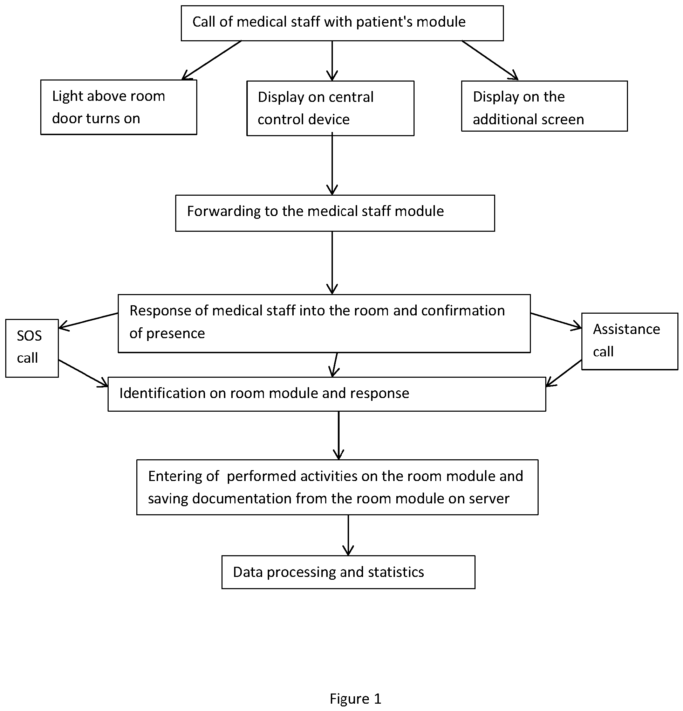

Figure 1 shows a scheme of the procedure for nurse call and management of health care and nursing, the said procedure comprising the following steps:
- calling medical staff with the button on the patient module, which may also be a bracelet or a pendant,
- alerting medical staff about the call via the central control device and/or the device for medical staff, optionally the call may also be displayed on the display screen,
- lighting of the notifying light above the door of the room, where the call has been made,
- identification of responsible medical staff on the room module for confirming presence with card identification, PIN code or any other known identification method,
- aiding the person who called or any other person, if the calling person has called to help someone else,
- possible call of additional staff, doctor, emergency team, wherein in case of the latter the notifying light emits blue light,
- entering performed activities in the room on the room module and/or device for medical staff,
- overview of possible other activities, particularly maintenance or cleaning,
- logging out on the room module before leaving the room,
- storing data on the server and possible analysis of response times, treatment time, performed activities and similar.

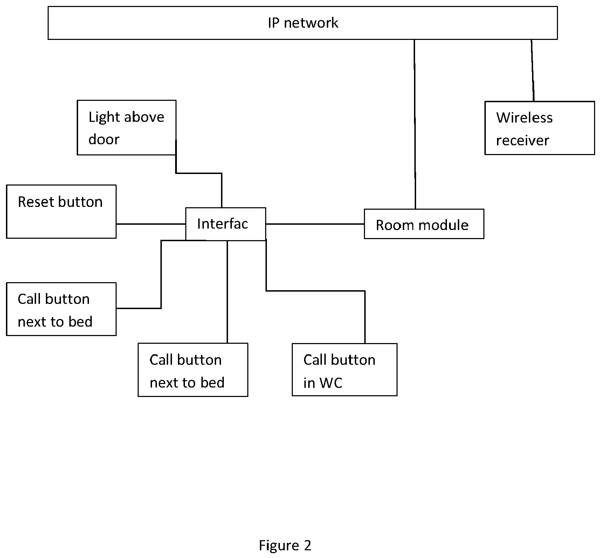

Figure 2 shows a possible embodiment of the system, where all units are connected with an IP network. The room module is via an interface connected with the light above the doors of the room, with the button for cancelling the call, with several patient's devices with call buttons. The interface is suitably arranged to enable response of the room module to a call, while the room module is arranged to transmit the information onto the central control device, to the devices for medical staff and to store information on the system server. The system may comprise several connected room modules and an arbitrary number of patient devices and an arbitrary number of devices for medical staff as well as supporting staff.

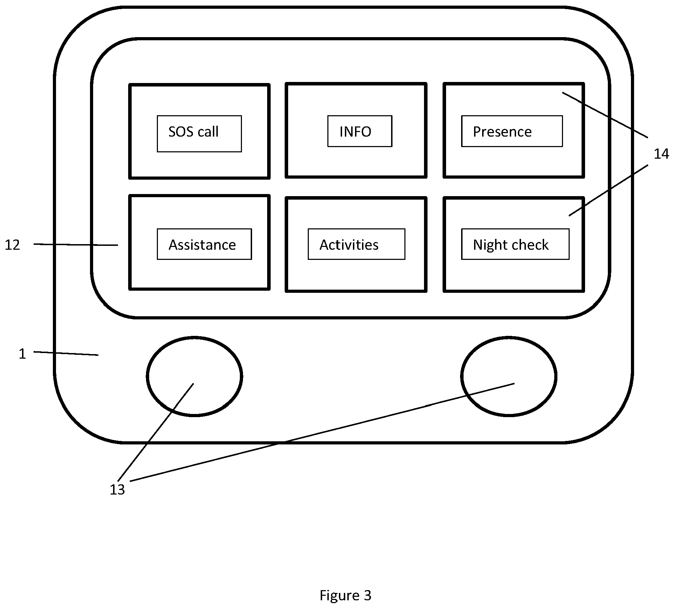

The room module 1 is shaped as a touch-screen display screen 12 with loudspeakers 13, having a menu with several options 14, preferably those shown in figure 3, which are:
- SOS call,
- Call of additional staff (Assistance call),
- information,
- presence,
- night inspection, and
- maintenance,
wherein the sub-menus information, maintenance and night inspection have several sub-choices, particularly the sub-menu information, in which all performed activities with patients in the room are entered.

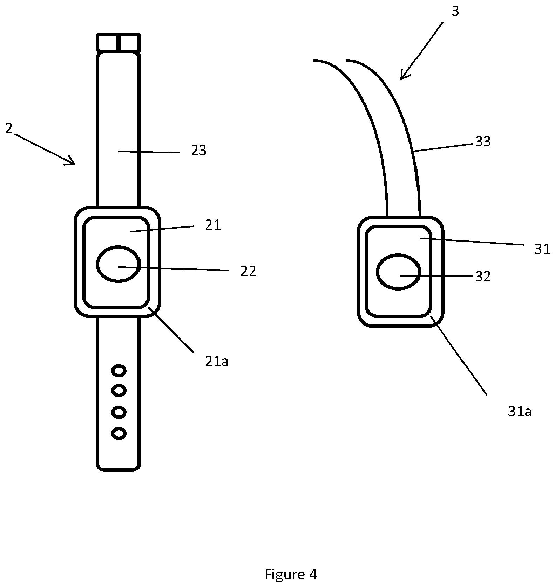

The room module also allows connection of patient devices, which are not located in a room, but the patient or a person wears them outside the room or even outside the institution. Such devices must have a transmitter 21, 31 and a button 22, 32 for calling medical staff, so that the module 2, 3 can sense the call and recognize location of the person with the activated device. Preferably such devices are shaped as a bracelet 2 on an adjustable strap 23, wherein the transmitter 21 is installed in a housing 21a, or a pendant 3 as shown in figure 4, which has a string 33 and a housing 31a of the transmitter 31 with a button 32.

## Claims

1. A system for a nurse call and management of health care and nursing comprises the following suitably interconnected devices, units or modules:
- at least one device in a room or any other space for nurse call and management of health care and nursing (i.e. room module),
- at least one user device for installation in a bathroom, above or beside a bed in a room of a healthcare institution, said device having at least a button for calling medical staff (i.e. patient module),
- at least one central control device for installation in a room for medical staff, which tracks calls from patient modules and stores information from the room module to a server of the system,
- at least one device for medical staff, which can connect to the room module and/or the central control device,
- at least one notifying lights for installation above room door,
- at least one suitable interface, which enables connection of all or a part of patient modules installed in any space wherein the interface has enough spaces for input and output signals,
wherein identification and use of the room module and the central control unit enables card identification, code indentification, preferably with PIN numbers, or any other suitable similar identification means; and
wherein the system performs at least the following operations:
- enabling calling of nursing staff from any patient module,
- enabling cancelling of nurse call on the room module, on the device for medical staff and/or on the central control unit,
- identification of staff or other employees on the room module,
- calling a doctor and/or emergency team via the room module or the device for medical staff,
- showing calls and emergency with the lights above doors and the central control device,
- enabling assistance call (=calling additional medical/nursing staff) via the room module or the device for medical staff,
- confirming performed checks,
- entering patient's data, status of the patient and consumed medications, food and/or drink and performed therapy,
- logging events and storing them on the system server,
and the operation of the system is supported with a computer program or protocols, which ensure suitable performance and logging operations as well as communication of all devices included in the system.

2. The system according to claim 1, **characterized in that** health technicians and caretakers, physiotherapists, occupational therapists, medical doctors and other health workers can check-in in the room module in the same way as nurses.

3. The system according to any of the preceding claims, **characterized in that** in addition to call button the system has a button for cancelling a call, which is suitably connected to the room module via an interface.

4. The system according to any of the preceding claims, **characterized in that** it has at least one device for cleaning staff and other employees of the health institution, which is preferably a mobile phone, a smart phone, a tablet or a similar device; and **in that** the system further performs at least the following operations:
- calling or notifying maintenance staff with a notification about needed repair,
- calling or notifying cleaning staff with a notification about needed cleaning,
- identification of supporting staff on the room module, wherein they cannot access to patient information and can only enter information about maintenance or cleaning,
- entering and storing of performed tasks on the system server.

5. The system according to any of the preceding claims, **characterized in that** the ceiling display of events connected with the central control device in case of a call shows the room number and optionally the name or number of the patient who made the call.

6. The system according to any of the preceding claims, **characterized in that** the components of the system are interconnected electronically, wherein they can communicate via wired networks or wireless networks, particularly via Bluetooth network, preferably via mesh Bluetooth network.

7. The system according to any of the preceding claims, **characterized in that** the room module is an interactive touch-screen display having a display, power system, connection module for wired or wireless connection, preferably Bluetooth, and all other component needed for operation, wherein the room module is connected to the central control unit and preferably to the system server; and enables access to at least the following information:
- staff member logged in the room,
- arrival into the room and time spent in the room,
- performed activities in the room, including performed measurements and results,
- the person for which the activities were performed,
wherein all enterings are made directly on the room module or via the device for medical staff.

8. The system according to the preceding claim, **characterized in that** da ima meni z različnimi možnostmi, prednostno:
- SOS call,
- call of additional staff (Assistance call),
- information,
- presence,
- night inspection, and
- maintenance,
wherein the sub-menues information, maintenance and night inspection have several sub-choices, particularly the sub-menu information, in which all performed activities with patients in the room are entered.

9. The system according to any of the preceding claims, **characterized in that** the device for medical staff is preferably designed as a smart phone, a tablet or a similar device arranged to operate a computer program and/or a mobile application, which enables overview of the present staff and setting up an assistance call, so that the staff may communicate without the need for a SIM card; and **in that** the device for medical staff allows communication with the room module or the central control unit, respectively, so that data about performed activities, results of performed measurements, data about consumed food, drink and/or medication, etc., is stored.

10. The system according to any of the preceding claims, **characterized in that** the patient module is equipped at least with a button for calling medical staff and a LED indicator of a triggered call, and optionally has additional functional buttons for controlling lights above the bed, TV, radio, and speech communication with nursing staff.

11. The system according to any of the preceding claims, **characterized in that** the patient module is shaped as a wireless pendant or a wireless bracelet, wherein it has a transmitter with a button for calling help and can connect to a central control unit of the system, as well as a sensor for sensing wear, preferably also a locating system provided for determination of patient's location in case he is not in a room, wherein the transmitter is arranged for performing an automatic reset of the call, so that after a triggered call via the patient module the responsive medical staff has a special emitter emitting a weak signal; upon reaching required distance of the medical staff to the patient, the patient's module senses the signal and sends call cancellation to the system.

12. The system according to any of the preceding claims, **characterized in that** operation of the device enables a suitable computer program and/or a mobile application, which performs processes for user identification, entering performed activities and data about the treated person, wherein the program ensures storing time stamps of arrival and departure, which enables tracking of treatment times as well as identification of presence in the room in time of performed activities and possible values of performed measurements, consumed food, drink and/or medication.

13. The system according to the preceding claim, **characterized in that** the computer program performs at least the following functions:
- enabling calling of nursing staff from any patient module,
- enabling cancelling of nurse call on the room module, on the device for medical staff and/or on the central control unit,
- identification of staff or other employees on the room module,
- calling a doctor and/or emergency team via the room module or the device for medical staff,
- showing calls and emergency with the lights above doors and the central control device,
- enabling assistance call (=calling additional medical/nursing staff) via the room module or the device for medical staff,
- confirming performed checks,
- entering patient's data, status of the patient and consumed medications, food and/or drink and performed therapy,
- logging events and storing them on the system server,
- full support of operation for room modules and storing information in a database,
- directing calls with regards to set parameters (time of day, department, call type, etc.) and
- preferably enabling manual or automatic reports of collected data.

14. The system according to any of the preceding claims, **characterized in that** it is installed in existing or new buildings where medical care or control of patients is necessary, for example hospitals, health centres, health resorts, and nursing homes for elderly.

15. A procedure for a nurse call and management of health care and nursing with the system according to any of the preceding claims, wherein the procedure comprises at least the following steps:
- calling medical staff with the button on the patient module, which may also be a bracelet or a pendant,
- alerting medical staff about the call via the central control device and/or the device for medical staff, optionally the call may also be displayed on the display screen,
- lighting of the notifying light above the door of the room, where the call has been made,
- identification of responsible medical staff on the room module for confirming presence with card identification, PIN code or any other known identification method,
- aiding the person who called or any other person, if the calling person has called to help someone else,
- possible call of additional staff, doctor, emergency team, wherein in case of the latter the notifying light emits blue light,
- entering performed activities in the room on the room module and/or device for medical staff,
- overview of possible other activities, particularly maintenance or cleaning,
- logging out on the room module before leaving the room,
- storing data on the server and possible analysis of response times, treatment time, performed activities and similar.
